# EUROPEAN PATENT APPLICATION

(11) **EP 3 757 121 A1**
(43) Date of publication of application: **30.12.2020**
(21) Application number: 19183411.8
(22) Date of filing: 28.06.2019
(51) Int. Cl.: C07K 14/775, A61P 25/28, G01N 33/68

(54) **APOLIPOPROTEIN E FRAGMENTS**

(71) Applicant: Eisai R&D Management Co., Ltd., Tokyo 112-8088 (JP); BioArctic AB, 112 51 Stockholm (SE)
(72) Inventor: HAGIWARA, Hiroaki, Tsukuba-shi Ibaraki 300-2635 (JP); HORIE, Kanta, Tsukuba-shi Ibaraki 300-2635 (JP); KANATSU, Kunihiko, Tsukuba-shi Ibaraki 300-2635 (JP); ISHIHARA, Yasuharu, Tsukuba-shi Ibaraki 300-2635 (JP); GOTO, Yasuaki, Tsukuba-shi Ibaraki 300-2635 (JP); OKI, Toru, Tsukuba-shi Ibaraki 300-2635 (JP); TSUBOI, Masafumi, Tsukuba-shi Ibaraki 300-2635 (JP); SAHLIN, Charlotte, SE-112 51 Stockholm (SE); ERIKSSON, Maria, SE-112 51 Stockholm (SE); MÖLLER, Christer, SE-112 51 Stockholm (SE)
(74) Representative: Boult Wade Tennant LLP

(57) **Abstract**

The present invention relates to novel fragments of apolipoprotein E (ApoE). These ApoE fragments have a variety of uses including as components of vaccine compositions, particularly vaccines for the prevention or treatment of neurological disorders such as Alzheimer's disease. The ApoE fragments may also be used in screening methods and methods of detection.

SEQUENCE LISTING

<110>  Eisai R&D Management Co., Ltd.
       BioArctic AB

<120>  APOLIPOPROTEIN E FRAGMENTS

<130>  NLW/P207532EP00

<160>  6

<170>  PatentIn version 3.5

<210>  1
<211>  100
<212>  PRT
<213>  Artificial

<220>
<223>  Synthetic peptide

<400>  1

Gly Gln Pro Leu Gln Glu Arg Ala Gln Ala Trp Gly Glu Arg Leu Arg
1               5                   10                  15

Ala Arg Met Glu Glu Met Gly Ser Arg Thr Arg Asp Arg Leu Asp Glu
            20                  25                  30

Val Lys Glu Gln Val Ala Glu Val Arg Ala Lys Leu Glu Glu Gln Ala
        35                  40                  45

Gln Gln Ile Arg Leu Gln Ala Glu Ala Phe Gln Ala Arg Leu Lys Ser
    50                  55                  60

Trp Phe Glu Pro Leu Val Glu Asp Met Gln Arg Gln Trp Ala Gly Leu
65                  70                  75                  80

Val Glu Lys Val Gln Ala Ala Val Gly Thr Ser Ala Ala Pro Val Pro
                85                  90                  95

Ser Asp Asn His
            100

<210>  2
<211>  101
<212>  PRT
<213>  Artificial

<220>
<223>  Synthetic peptide

<400>  2

Ala Gly Gln Pro Leu Gln Glu Arg Ala Gln Ala Trp Gly Glu Arg Leu
1               5                   10                  15

Arg Ala Arg Met Glu Glu Met Gly Ser Arg Thr Arg Asp Arg Leu Asp
            20                  25                  30

Glu Val Lys Glu Gln Val Ala Glu Val Arg Ala Lys Leu Glu Glu Gln
        35                  40                  45

Ala Gln Gln Ile Arg Leu Gln Ala Glu Ala Phe Gln Ala Arg Leu Lys
    50                  55                  60

Ser Trp Phe Glu Pro Leu Val Glu Asp Met Gln Arg Gln Trp Ala Gly
65                  70                  75                  80

Leu Val Glu Lys Val Gln Ala Ala Val Gly Thr Ser Ala Ala Pro Val
                85                  90                  95

Pro Ser Asp Asn His
            100

<210> 3
<211> 102
<212> PRT
<213> Artificial

<220>
<223> Synthetic peptide

<400> 3

Leu Ala Gly Gln Pro Leu Gln Glu Arg Ala Gln Ala Trp Gly Glu Arg
1               5                   10                  15

Leu Arg Ala Arg Met Glu Glu Met Gly Ser Arg Thr Arg Asp Arg Leu
            20                  25                  30

Asp Glu Val Lys Glu Gln Val Ala Glu Val Arg Ala Lys Leu Glu Glu
        35                  40                  45

Gln Ala Gln Gln Ile Arg Leu Gln Ala Glu Ala Phe Gln Ala Arg Leu
    50                  55                  60

Lys Ser Trp Phe Glu Pro Leu Val Glu Asp Met Gln Arg Gln Trp Ala
65                  70                  75                  80

Gly Leu Val Glu Lys Val Gln Ala Ala Val Gly Thr Ser Ala Ala Pro
                85                  90                  95

Val Pro Ser Asp Asn His
            100

<210>  4
<211>  299
<212>  PRT
<213>  Homo sapiens

<400>  4

Lys Val Glu Gln Ala Val Glu Thr Glu Pro Glu Pro Glu Leu Arg Gln
1               5                   10                  15

Gln Thr Glu Trp Gln Ser Gly Gln Arg Trp Glu Leu Ala Leu Gly Arg
            20                  25                  30

Phe Trp Asp Tyr Leu Arg Trp Val Gln Thr Leu Ser Glu Gln Val Gln
        35                  40                  45

Glu Glu Leu Leu Ser Ser Gln Val Thr Gln Glu Leu Arg Ala Leu Met
    50                  55                  60

Asp Glu Thr Met Lys Glu Leu Lys Ala Tyr Lys Ser Glu Leu Glu Glu
65                  70                  75                  80

Gln Leu Thr Pro Val Ala Glu Glu Thr Arg Ala Arg Leu Ser Lys Glu
                85                  90                  95

Leu Gln Ala Ala Gln Ala Arg Leu Gly Ala Asp Met Glu Asp Val Cys
            100                 105                 110

Gly Arg Leu Val Gln Tyr Arg Gly Glu Val Gln Ala Met Leu Gly Gln
        115                 120                 125

Ser Thr Glu Glu Leu Arg Val Arg Leu Ala Ser His Leu Arg Lys Leu
    130                 135                 140

Arg Lys Arg Leu Leu Arg Asp Ala Asp Asp Leu Gln Lys Cys Leu Ala
145                 150                 155                 160

Val Tyr Gln Ala Gly Ala Arg Glu Gly Ala Glu Arg Gly Leu Ser Ala
                165                 170                 175

Ile Arg Glu Arg Leu Gly Pro Leu Val Glu Gln Gly Arg Val Arg Ala
            180                 185                 190

Ala Thr Val Gly Ser Leu Ala Gly Gln Pro Leu Gln Glu Arg Ala Gln
        195                 200                 205

Ala Trp Gly Glu Arg Leu Arg Ala Arg Met Glu Glu Met Gly Ser Arg
    210                 215                 220

Thr Arg Asp Arg Leu Asp Glu Val Lys Glu Gln Val Ala Glu Val Arg
225                 230                 235                 240

Ala Lys Leu Glu Glu Gln Ala Gln Gln Ile Arg Leu Gln Ala Glu Ala
                245                 250                 255

Phe Gln Ala Arg Leu Lys Ser Trp Phe Glu Pro Leu Val Glu Asp Met
            260                 265                 270

Gln Arg Gln Trp Ala Gly Leu Val Glu Lys Val Gln Ala Ala Val Gly
        275                 280                 285

Thr Ser Ala Ala Pro Val Pro Ser Asp Asn His
    290                 295

<210>  5
<211>  299
<212>  PRT
<213>  Homo sapiens

<400>  5

Lys Val Glu Gln Ala Val Glu Thr Glu Pro Glu Pro Glu Leu Arg Gln
1               5                   10                  15

Gln Thr Glu Trp Gln Ser Gly Gln Arg Trp Glu Leu Ala Leu Gly Arg
            20                  25                  30

Phe Trp Asp Tyr Leu Arg Trp Val Gln Thr Leu Ser Glu Gln Val Gln
        35                  40                  45

Glu Glu Leu Leu Ser Ser Gln Val Thr Gln Glu Leu Arg Ala Leu Met
    50                  55                  60

Asp Glu Thr Met Lys Glu Leu Lys Ala Tyr Lys Ser Glu Leu Glu Glu
65                  70                  75                  80

Gln Leu Thr Pro Val Ala Glu Glu Thr Arg Ala Arg Leu Ser Lys Glu
                85                  90                  95

Leu Gln Ala Ala Gln Ala Arg Leu Gly Ala Asp Met Glu Asp Val Cys
            100                 105                 110

Gly Arg Leu Val Gln Tyr Arg Gly Glu Val Gln Ala Met Leu Gly Gln
        115                 120                 125

Ser Thr Glu Glu Leu Arg Val Arg Leu Ala Ser His Leu Arg Lys Leu
    130                 135                 140

Arg Lys Arg Leu Leu Arg Asp Ala Asp Asp Leu Gln Lys Arg Leu Ala
145                 150                 155                 160

Val Tyr Gln Ala Gly Ala Arg Glu Gly Ala Glu Arg Gly Leu Ser Ala
                165                 170                 175

Ile Arg Glu Arg Leu Gly Pro Leu Val Glu Gln Gly Arg Val Arg Ala
            180                 185                 190

Ala Thr Val Gly Ser Leu Ala Gly Gln Pro Leu Gln Glu Arg Ala Gln
        195                 200                 205

Ala Trp Gly Glu Arg Leu Arg Ala Arg Met Glu Glu Met Gly Ser Arg
    210                 215                 220

Thr Arg Asp Arg Leu Asp Glu Val Lys Glu Gln Val Ala Glu Val Arg
225                 230                 235                 240

Ala Lys Leu Glu Glu Gln Ala Gln Gln Ile Arg Leu Gln Ala Glu Ala
                245                 250                 255

Phe Gln Ala Arg Leu Lys Ser Trp Phe Glu Pro Leu Val Glu Asp Met
            260                 265                 270

Gln Arg Gln Trp Ala Gly Leu Val Glu Lys Val Gln Ala Ala Val Gly
        275                 280                 285

Thr Ser Ala Ala Pro Val Pro Ser Asp Asn His
    290                 295

<210>  6
<211>  299
<212>  PRT
<213>  Homo sapiens

<400>  6

Lys Val Glu Gln Ala Val Glu Thr Glu Pro Glu Pro Glu Leu Arg Gln
1               5                   10                  15

Gln Thr Glu Trp Gln Ser Gly Gln Arg Trp Glu Leu Ala Leu Gly Arg
            20                  25                  30

Phe Trp Asp Tyr Leu Arg Trp Val Gln Thr Leu Ser Glu Gln Val Gln
        35                  40                  45

Glu Glu Leu Leu Ser Ser Gln Val Thr Gln Glu Leu Arg Ala Leu Met
    50                  55                  60

Asp Glu Thr Met Lys Glu Leu Lys Ala Tyr Lys Ser Glu Leu Glu Glu
65                  70                  75                  80

Gln Leu Thr Pro Val Ala Glu Glu Thr Arg Ala Arg Leu Ser Lys Glu
                85                  90                  95

Leu Gln Ala Ala Gln Ala Arg Leu Gly Ala Asp Met Glu Asp Val Arg
            100                 105                 110

Gly Arg Leu Val Gln Tyr Arg Gly Glu Val Gln Ala Met Leu Gly Gln
        115                 120                 125

Ser Thr Glu Glu Leu Arg Val Arg Leu Ala Ser His Leu Arg Lys Leu
    130                 135                 140

Arg Lys Arg Leu Leu Arg Asp Ala Asp Asp Leu Gln Lys Arg Leu Ala
145                 150                 155                 160

Val Tyr Gln Ala Gly Ala Arg Glu Gly Ala Glu Arg Gly Leu Ser Ala
                165                 170                 175

Ile Arg Glu Arg Leu Gly Pro Leu Val Glu Gln Gly Arg Val Arg Ala
            180                 185                 190

Ala Thr Val Gly Ser Leu Ala Gly Gln Pro Leu Gln Glu Arg Ala Gln
        195                 200                 205

Ala Trp Gly Glu Arg Leu Arg Ala Arg Met Glu Glu Met Gly Ser Arg
    210                 215                 220

Thr Arg Asp Arg Leu Asp Glu Val Lys Glu Gln Val Ala Glu Val Arg
225                 230                 235                 240

Ala Lys Leu Glu Glu Gln Ala Gln Gln Ile Arg Leu Gln Ala Glu Ala
                245                 250                 255

Phe Gln Ala Arg Leu Lys Ser Trp Phe Glu Pro Leu Val Glu Asp Met
            260                 265                 270

Gln Arg Gln Trp Ala Gly Leu Val Glu Lys Val Gln Ala Ala Val Gly
        275                 280                 285

Thr Ser Ala Ala Pro Val Pro Ser Asp Asn His
    290                 295

## Description

### Field of the invention

The present invention relates to novel fragments of apolipoprotein E (ApoE). These ApoE fragments have a variety of uses including as components of vaccine compositions, particularly vaccines for the prevention or treatment of neurological disorders such as Alzheimer's disease. The ApoE fragments may also be used in screening methods and methods of detection as described herein.

### Background

Apolipoprotein E (ApoE) is a protein that plays a central role in lipoprotein metabolism through its high-affinity binding to the low density lipoprotein (LDL) receptor family. ApoE circulates in the blood and is also found associated with high density lipoproteins in the cerebrospinal fluid and central nervous system interstitial fluid.

Full-length human ApoE is a 34 kDa protein consisting of two domains. The N-terminal domain (residues 1-191) is primarily responsible for the LDL-receptor binding activity of ApoE whilst the C-terminal domain (residues 216-299) binds to lipoprotein with high affinity.

ApoE exists in three different isoforms, ApoE2, ApoE3 and ApoE4, encoded by the APOE ε2, ε3 and ε4 alleles, respectively. The APOE ε4 allele is the strongest known genetic risk factor for late-onset Alzheimer's disease (AD).

Alzheimer's disease (AD) is a progressive neurodegenerative dementia disorder, which exists in a more common late-onset form and an early-onset familial form. AD is characterized by progressive loss of memory and cognitive function. At present, AD treatments are limited to symptomatic management and the prognosis is poor for AD patients. It is estimated that about 18 million people worldwide are presently suffering from AD, and the number of people suffering from AD is expected to increase due to the aging population. The prevalence of AD doubles approximately every 5 years from the age of 60, from 10% of individuals at the age of 65 to 50% of individuals at the age of 85 or more (Solomon, Expert Opin. Investig. Drugs (2007) 16(6): 819-828).

The different mechanisms by which ApoE4 is thought to contribute to AD and the pathology of other neurological disorders are reviewed in Mahley and Huang (Neuron (2012) 76: 871-885). These mechanisms include effects at the level of amyloid plaque formation including the regulation of amyloid β (Aβ) metabolism, clearance, aggregation and deposition. ApoE4 and fragments thereof have been reported to bind to Aβ thereby directly affecting Aβ turnover and the formation of Aβ fibrils (Garai et al. Biochemistry (2014) 53: 6323-6331; Jones et al. PLoS ONE (2011) 6(1): e14586; Mouchard et al. Sci. Rep. (2019) 9(1): 3989; and Wellnitz et al. J. Neurochem. (2005) 94: 1351-1360).

ApoE has also been reported as having a direct role in causing neuropathology. Under normal physiological conditions, ApoE in the brain is synthesized primarily by astrocytes to support lipid transport and membrane repair processes. However, in response to neuronal insult or injury, ApoE is synthesized by neurons. The ApoE produced by neurons is susceptible to proteolysis and studies have revealed the accumulation of neurotoxic C-terminal truncated fragments generated by a chymotrypsin-like serine protease (Harris et al. PNAS (2003) 100(19): 10966-10971).

Further characterization of these C-terminal fragments revealed that an ApoE4(1-272) fragment caused mitochondrial dysfunction and was neurotoxic but that full-length ApoE4(1-299) and a shorter fragment ApoE4(1-240) did not bring about these effects. In addition, truncation of the N-terminal region (1-170) containing the LDL receptor binding region (amino acids 135-150) abolished the effects seen with the ApoE4(1-272) fragment indicating that the N-terminal receptor binding region and C-terminal lipid binding region of ApoE act in concert to cause mitochondrial dysfunction and neurotoxicity (Chang et al. PNAS (2005) 102(51): 18694-18699).

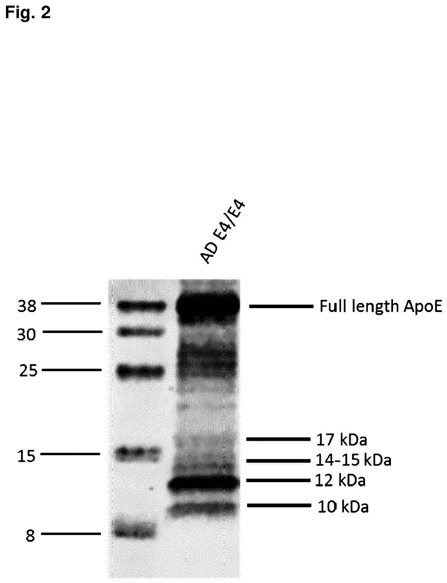

Studies have revealed the presence of ApoE fragments in the brains and cerebrospinal fluid from humans with AD and recently, the role of ApoE fragments in AD has been reviewed by Muñoz et al in Neurochem Res (2019) 44(6):1297-1305. The majority of ApoE fragments described in Muñoz *et al* are N-terminal fragments of the protein. However, with regards to ApoE fragments from the C-terminal lipid-binding domain, Table 2 and Figure 2 of Muñoz *et al* indicate that one such fragment has been studied previously and shown to have a stabilizing effect on hexamers of Aβ peptide. The study in question was reported by Wellnitz et al in J Neurochem (2005) 94: 1351-1360, and describes a 13 kDa fragment of ApoE with an N-terminal start at amino acid position 187 of ApoE.

More recently, a study by Mouchard *et al* (Sci Rep (2019) 9(1): 3989) reported the identification of ApoE fragments in the post-mortem brains of ADe patients. This study reported the presence of 12 kDa, 16 kDa and 18 kDa ApoE forms present in the cortex of AD patients. Only the 18 kDa fragment was found to be significantly increased in AD patients. The 16 kDa and 18 kDa forms of ApoE lacking both the NH2 -half and the C-terminal end of ApoE were found to associate with Aβ and were proposed as mediators of AD pathology. In contrast, the small 12 kDa fragments of ApoE were not found to bind Aβ.

### Summary of the invention

It is clear that ApoE plays a key role in the pathology of a variety of neurological disorders, particularly neurodegenerative conditions such as Alzheimer's disease (AD). As such, there is a need to understand the biology of this protein so as to formulate effective therapeutic strategies. The present application reports the identification of novel ApoE fragments in brain tissue obtained from AD patients.

Thus, in a first aspect, provided herein is a fragment of apolipoprotein E (ApoE), which consists of an amino acid sequence selected from the group consisting of SEQ ID NO: 2 and SEQ ID NO: 3.

Further encompassed are: isolated nucleic acids encoding the ApoE fragments; vectors comprising the isolated nucleic acids; and host cells and transgenic non-human animals comprising the vectors.

In a second aspect, provided herein is a vaccine composition comprising an apolipoprotein E (ApoE) fragment consisting of the amino acid sequence of any one of SEQ ID NOs: 1-3. Further provided are methods of preventing or treating a neurological disease in a subject, particularly a neurodegenerative disease, wherein the methods comprise administering to the subject an ApoE vaccine. In preferred embodiments, the vaccine is administered so as to prevent or treat Alzheimer's disease.

In a further aspect, provided herein is a method of screening for a pharmacological agent having the ability to modulate the neuronal toxicity of an apolipoprotein E fragment consisting of the amino acid sequence of any one of SEQ ID NOs: 1-3, wherein the method comprises contacting a neural cell or non-human animal with a candidate pharmacological agent in the presence of the fragment and detecting neuronal toxicity or neuronal death.

In a further aspect, provided herein is a method of screening for a pharmacological agent having the ability to modulate the production of an apolipoprotein E fragment consisting of the amino acid sequence of any one of SEQ ID NOs: 1-3, wherein the method comprises contacting a neural cell expressing apolipoprotein E with a candidate pharmacological agent and detecting the amount of the fragment.

In a further aspect, provided herein is a method for detecting the presence or amount of an apolipoprotein E fragment consisting of the amino acid sequence of any one of SEQ ID NOs: 1-3 in a subject, wherein the method comprises contacting a sample obtained from the subject with an aptamer that binds to the fragment and detecting the presence or the amount of the fragment in the sample.

### Brief description of the figures

Figure 1 shows the results of Western blot analysis of human brain extracts as described in Example 1.
Figure 2 shows the results of Western blot analysis of human brain extract from AD brain of genotype APOE ε4/ε4 at sufficiently high resolution to show individual low molecular weight ApoE fragments as described in Example 1.
Figure 3 is a diagram showing the ratio of 12 kDa ApoE fragment to full-length ApoE in AD (filled circles) and control (open squares), quantified as described in Example 1.
Figure 4 is a diagram showing the ratio of 12 kDa ApoE fragment to full-length ApoE in AD without APOE E4 genotype (-E4; filled circles) or with APOE E4 genotype (+E4; open squares), quantified as described in Example 1.
Figure 5 is a schematic overview of the workflow for the immunoprecipitation experiments described in Example 2.
Figure 6 shows the result of Western blot analysis of immunoprecipitated samples as described in Example 2.
Figure 7 shows the result of silver staining of immunoprecipitated samples as described in Example 2.
Figure 8 shows the result of LC-MS/MS analysis of tryptic digests of 12 kDa, 15 kDa and rhApoE4 gels as indicated, as described in Example 3.
Figure 9 shows the result of LysC cleavage site analysis of the ApoE sequence as described in Example 4.
Figure 10 shows the result of investigation by extracted-ion chromatograms (XIC) of theoretical ApoE cleavage sites as described in Example 5. Left side: Extracted ion chromatograms at theoretical values of three charge states of one of the possible peptides (200-233) with 5 ppm mass accuracy, with peaks observed at the same retention time for all three. Right side: The mass spectrum from each extracted peak.
Figure 11 shows the result of nanoLC-MS/MS with the shotgun proteomic method for detection of peptides around cleavage sites as described in Example 5. In replicate analyses of samples from the same donor (ApoE ε3/ε4, A and B), peptides having an N terminus at 198L, 199A or 200G and an intact C terminus of ApoE were detected.
Figure 12 is a diagram showing the MS intensity for peptides having an N terminus at 198L, 199A or 200G in samples from ApoE ε4/ε4, ε2/ε3 and ε3/ε3 carriers as indicated, as described in Example 6.
Figure 13 shows the mitochondrial damages induced by human ApoE4 and ApoE C-terminal fragments following the experiment described in Example 7, in (A) Neuro2A cells and (B) rat primary hippocampal neurons; as well as (C) protein expression of human ApoE4 or ApoE C-terminal fragments as measured by Western blot analysis.

### Detailed Description

### ApoE Fragments

The present disclosure is directed to fragments of apolipoprotein E (ApoE). As reported herein, ApoE fragments are significantly increased in Alzheimer's disease (AD) patients, particularly AD patients having the APOE ε4 allele.

The ApoE fragments of the disclosure are derived from the C-terminus of the full-length human ApoE protein. The full-length human ApoE proteins are shown in Table 1 below (ApoE2, ApoE3 and ApoE4). Also shown in Table 1 are the C-terminal fragments consisting of amino acids 200-299 (SEQ ID NO: 1), amino acids 199-299 (SEQ ID NO: 2) and amino acids 198-299 (SEQ ID NO: 3) of human ApoE. As is evident from the full-length sequences of the different ApoE isoforms, the C-terminal fragments (as represented by SEQ ID NOs: 1-3) are common to all isoforms. As such, the ApoE fragments described herein may be produced or found in individuals having any of the APOE alleles selected from ε2, ε3 and ε4. As reported herein, the ApoE fragments are found at higher levels in AD patients having at least one ε4 allele.

**Table 1**

| SEQ ID NO: | Description | Sequence |
|---|---|---|
| 1 | ApoE 200-299 | GQPLQERAQAWGERLRARMEEMGSRTRDRLDEVKEQVAE VRAKLEEQAQQIRLQAEAFQARLKSWFEPLVEDMQRQWAG LVEKVQAAVGTSAAPVPSDNH |
| 2 | ApoE 199-299 | AGQPLQERAQAWGERLRARMEEMGSRTRDRLDEVKEQVA EVRAKLEEQAQQIRLQAEAFQARLKSWFEPLVEDMQRQWA GLVEKVQAAVGTSAAPVPSDNH |
| 3 | ApoE 198-299 | LAGQPLQERAQAWGERLRARMEEMGSRTRDRLDEVKEQVA EVRAKLEEQAQQIRLQAEAFQARLKSWFEPLVEDMQRQWA GLVEKVQAAVGTSAAPVPSDNH |
| 4 | Human ApoE2 | KVEQAVETEPEPELRQQTEWQSGQRWELALGRFWDYLRW VQTLSEQVQEELLSSQVTQELRALMDETMKELKAYKSELEE QLTPVAEETRARLSKELQAAQARLGADMEDVCGRLVQYRGE VQAMLGQSTEELRVRLASHLRKLRKRLLRDADDLQKCLAVY QAGAREGAERGLSAIRERLGPLVEQGRVRAATVGSLAGQPL QERAQAWGERLRARMEEMGSRTRDRLDEVKEQVAEVRAKL EEQAQQIRLQAEAFQARLKSWFEPLVEDMQRQWAGLVEKV QAAVGTSAAPVPSDNH |
| 5 | Human ApoE3 | KVEQAVETEPEPELRQQTEWQSGQRWELALGRFWDYLRW VQTLSEQVQEELLSSQVTQELRALMDETMKELKAYKSELEE QLTPVAEETRARLSKELQAAQARLGADMEDVCGRLVQYRGE VQAMLGQSTEELRVRLASHLRKLRKRLLRDADDLQKRLAVY QAGAREGAERGLSAIRERLGPLVEQGRVRAATVGSLAGQPL QERAQAWGERLRARMEEMGSRTRDRLDEVKEQVAEVRAKL EEQAQQIRLQAEAFQARLKSWFEPLVEDMQRQWAGLVEKV QAAVGTSAAPVPSDNH |
| 6 | Human ApoE4 | KVEQAVETEPEPELRQQTEWQSGQRWELALGRFWDYLRW VQTLSEQVQEELLSSQVTQELRALMDETMKELKAYKSELEE QLTPVAEETRARLSKELQAAQARLGADMEDVRGRLVQYRGE VQAMLGQSTEELRVRLASHLRKLRKRLLRDADDLQKRLAVY QAGAREGAERGLSAIRERLGPLVEQGRVRAATVGSLAGQPL QERAQAWGERLRARMEEMGSRTRDRLDEVKEQVAEVRAKL EEQAQQIRLQAEAFQARLKSWFEPLVEDMQRQWAGLVEKV QAAVGTSAAPVPSDNH |

In one embodiment, provided herein is a fragment of apolipoprotein E (ApoE) consisting of the amino acid sequence of SEQ ID NO: 1. In certain embodiments, provided herein are ApoE fragments consisting of an amino acid sequence having at least 80%, at least 85%, at least 90%, at least 95%, at least 97%, at least 98%, at least 99% identity to SEQ ID NO: 1. In one embodiment, provided herein is a fragment of apolipoprotein E (ApoE) consisting of amino acids 200-299 of human ApoE.

In one embodiment, provided herein is a fragment of apolipoprotein E (ApoE) consisting of the amino acid sequence of SEQ ID NO: 2. In certain embodiments, provided herein are ApoE fragments consisting of an amino acid sequence having at least 80%, at least 85%, at least 90%, at least 95%, at least 97%, at least 98%, at least 99% identity to SEQ ID NO: 2. In one embodiment, provided herein is a fragment of apolipoprotein E (ApoE) consisting of amino acids 199-299 of human ApoE.

In one embodiment, provided herein is a fragment of apolipoprotein E (ApoE) consisting of the amino acid sequence of SEQ ID NO: 3. In certain embodiments, provided herein are ApoE fragments consisting of an amino acid sequence having at least 80%, at least 85%, at least 90%, at least 95%, at least 97%, at least 98%, at least 99% identity to SEQ ID NO: 3. In one embodiment, provided herein is a fragment of apolipoprotein E (ApoE) consisting of amino acids 198-299 of human ApoE.

As reported herein, the ApoE fragments exhibit neurotoxicity as measured *in vitro* by determining the respiratory capacity of neuronal cells in culture. Thus, in certain embodiments, the ApoE fragments described herein exhibit neurotoxicity. Neurotoxicity may be measured using any assay suitable for the detection of toxic effects in neuronal cells. Suitable assays are exemplified herein (see Example 7) and can be used to assess the neurotoxic properties of the ApoE fragments described herein.

The present disclosure also encompasses nucleic acids encoding the ApoE fragments described herein. Nucleic acids encoding the ApoE fragments include, for example, recombinant DNA molecules. The term "nucleic acid" is used herein interchangeably with "polynucleotide" or "polynucleotide molecule" and refers to any DNA or RNA molecule, either single- or double-stranded and, if single-stranded, the molecule of its complementary sequence. In discussing nucleic acids, a sequence or structure of a particular nucleic acid may be described according to the normal convention of providing the sequence in the 5' to 3' direction. In certain embodiments, the nucleic acid encodes an ApoE fragment consisting of the amino acid sequence of SEQ ID NO: 1. In certain embodiments, the nucleic acid encodes an ApoE fragment consisting of the amino acid sequence of SEQ ID NO: 2. In certain embodiments, the nucleic acid encodes an ApoE fragment consisting of the amino acid sequence of SEQ ID NO: 3.

In some embodiments, nucleic acids or polynucleotides are "isolated." This term, when applied to a nucleic acid, refers to a nucleic acid molecule that is separated from sequences with which it is immediately contiguous in the naturally occurring genome of the organism in which it originated. For example, an "isolated nucleic acid" may comprise a DNA molecule inserted into a vector, such as a plasmid or virus vector, or integrated into the genomic DNA of a prokaryotic or eukaryotic cell or non-human host organism. When applied to RNA, the term "isolated polynucleotide" refers primarily to an RNA molecule encoded by an isolated DNA molecule as defined above. Alternatively, the term may refer to an RNA molecule that has been purified/separated from other nucleic acids with which it would be associated in its natural state (i.e., in cells or tissues). An isolated polynucleotide (either DNA or RNA) may further represent a molecule produced directly by biological or synthetic means and separated from other components present during its production.

Also encompassed are vectors comprising the nucleic acids encoding the ApoE fragments. The vector may be a replicable vector suitable for expression of the ApoE fragment in a particular host cell or cell-free expression system. Vectors, including expression vectors suitable for use in a variety of different expression systems, are known in the art. Vectors incorporating nucleic acids encoding the ApoE fragments described herein may be prepared using any standard molecular biology techniques.

Vectors comprising the nucleic acids encoding the ApoE fragments may be incorporated into host cells. Suitable host cells may be prokaryote, yeast, or higher eukaryote cells, specifically mammalian cells. Examples of useful mammalian host cell lines are monkey kidney CV1 line transformed by SV40 (COS-7, ATCC CRL 1651); human embryonic kidney line (293 or 293 cells subcloned for growth in suspension culture, Graham et al., J. Gen. Virol. (1977) 36: 59); baby hamster kidney cells (BHK, ATCC CCL 10); Chinese hamster ovary cells/-DHFR (CHO, Urlaub et al., Proc. Natl. Acad. Sci. USA (1980) 77:4216); mouse sertoli cells (TM4, Mather, Biol. Reprod. (1980) 23: 243-251); mouse myeloma cells SP2/0-AG14 (ATCC CRL 1581; ATCC CRL 8287) or NS0 (HPA culture collections no. 85110503); monkey kidney cells (CV1 ATCC CCL 70); African green monkey kidney cells (VERO-76, ATCC CRL-1587); human cervical carcinoma cells (HELA, ATCC CCL 2); canine kidney cells (MDCK, ATCC CCL 34); buffalo rat liver cells (BRL 3A, ATCC CRL 1442); human lung cells (W138, ATCC CCL 75); human liver cells (Hep G2, HB 8065); mouse mammary tumor (MMT 060562, ATCC CCL51); TRI cells (Mather et al., Annals N.Y. Acad. Sci. 383:44-68 (1982)); MRC 5 cells; FS4 cells; and a human hepatoma line (Hep G2), as well as DSM's PERC-6 cell line.

It should be noted that the term "host cell" generally refers to a cultured cell line. Whole human beings into which an expression vector encoding an ApoE fragment has been introduced are explicitly excluded from the definition of a "host cell".

In certain embodiments, vectors comprising the nucleic acids encoding the ApoE fragments may be incorporated into transgenic non-human animals. Such animals may include but are not limited to mice, rats, rabbits, pigs.

The disclosure also encompasses methods of producing ApoE fragments described herein which methods comprise culturing a host cell (or cell free expression system) containing nucleic acid (e.g. an expression vector) encoding the ApoE fragment under conditions which permit expression of the fragment, and recovering the expressed fragment. This recombinant expression process can be used for large scale production of ApoE fragments, for example for use in vaccines or screening methods as described elsewhere herein. Suitable vectors, cell lines and production processes for large scale manufacture of recombinant polypeptides are generally available in the art and can be well known to the skilled person.

### Vaccines

The ApoE fragments described herein may be incorporated into vaccines, particularly vaccines for use in the prevention or treatment of neurological disorders or conditions, for example Alzheimer's disease.

In certain embodiments, the vaccine comprises one or more ApoE fragments and at least one adjuvant. In certain embodiments, the vaccine comprises the ApoE fragment consisting of the amino acid sequence of SEQ ID NO: 1 and at least one adjuvant. In certain embodiments, the vaccine comprises the ApoE fragment consisting of the amino acid sequence of SEQ ID NO: 2 and at least one adjuvant. In certain embodiments, the vaccine comprises the ApoE fragment consisting of the amino acid sequence of SEQ ID NO: 3 and at least one adjuvant. In certain embodiments, the vaccine comprises at least two or at least three ApoE fragments selected from SEQ ID NOs: 1, 2 and 3, and at least one adjuvant.

The vaccines or vaccine compositions may comprise two or more adjuvants. The purpose of the adjuvant(s) is to increase or stimulate the immune response in the subject. A variety of adjuvants are known in the art and may be used in the vaccines described herein. Particular adjuvants that may be employed include but are not limited to aluminium hydroxide (Alum) and/or CpG amongst others.

The vaccines may be used prophylactically i.e. they may be administered to subjects who are asymptomatic for disease so as induce an immune response aimed at preventing the development of a neurological disorder or condition. The vaccines may be used to immunize subjects so as to prevent the development of neurodegenerative diseases or disorders. The vaccines may be used to immunize subjects so as to prevent the development of diseases or disorders characterized by a loss of cognitive memory capacity. Such diseases or disorders include but are not limited to Alzheimer's disease (AD), mild cognitive impairment (MCI), dementia with Lewy body, Down's syndrome, and hereditary cerebral hemorrhage with amyloidosis (Dutch type). In certain embodiments, the vaccines may be used to prevent diseases or disorders associated with amylogenic proteins, such as cerebral amyloid angiopathy, Parkinson's disease, and cataract due to amyloid beta deposition. In preferred embodiments, the vaccines are used to prevent MCI or AD, preferably AD. The subject is typically a mammal and is preferably a human.

Alternatively or in addition, the vaccines may be used therapeutically i.e. they may be administered to subjects having a neurological disease or condition or suspected of having a neurological disease or condition so as to induce an immune response aimed at alleviating the symptoms associated with the disease. The vaccines may be used to treat neurodegenerative diseases or disorders. The vaccines may be used to treat diseases or disorders characterized by a loss of cognitive memory capacity. Such diseases or disorders include but are not limited to Alzheimer's disease (AD), mild cognitive impairment (MCI), dementia with Lewy body, Down's syndrome, and hereditary cerebral hemorrhage with amyloidosis (Dutch type). In certain embodiments, the vaccines may be used to treat diseases or disorders associated with amylogenic proteins, such as cerebral amyloid angiopathy, Parkinson's disease, and cataract due to amyloid beta deposition. In preferred embodiments, the vaccines are used to treat MCI or AD, preferably AD. The subject is typically a mammal and is preferably a human.

It follows from the above, that the present invention encompasses methods of preventing or treating a neurological disease or condition in a subject in need thereof, the methods comprising administering to the subject a vaccine comprising an ApoE fragment as described herein. In preferred embodiments, the methods are for the prevention or treatment of MCI and/or AD, preferably AD. Further provided herein is a vaccine in accordance with any of the embodiments described for use in the prevention or treatment of a neurological disease or condition in a subject in need thereof. In preferred embodiments, the vaccine is for use in the prevention or treatment of MCI and/or AD, preferably AD.

The vaccines may be administered to the subject by any appropriate route of administration. As the skilled person would be aware, vaccine compositions may be administered by topical, oral, rectal, nasal or parenteral (such as intravenous, intradermal, subcutaneous, or intramuscular) routes. In addition, vaccines may be incorporated into sustained release matrices such as biodegradable polymers, the polymers being implanted in the vicinity of, or in close proximity to, where delivery is desired. In preferred embodiments, the vaccine is administered intramuscularly or subcutaneously.

The vaccines may be administered a single time to the subject to generate an immune response. In some embodiments, the vaccines are administered multiple times to the same subject. Thus, so-called prime-boost regimens may be employed.

Further provided herein are kits containing vaccines as described herein. Such kits may be provided with suitable instructions for use. The instructions for use may explain the administration schedule for the vaccine. The kits may therefore comprise multiple (separate) doses of the vaccine for administration to a subject. The instructions for use may further explain the storage conditions for the vaccines, particularly during the time period between administration of the doses of the vaccines.

### Methods of screening

Further provided herein are methods of screening based upon the novel ApoE fragments described herein.

In one aspect, provided herein are methods of screening for pharmacological agents having the ability to modulate the neuronal toxicity of ApoE fragments wherein the ApoE fragments are selected from the fragments represented by any one of SEQ ID NOs: 1, 2 or 3. In certain embodiments, the methods of screening are used to identify pharmacological agents having the ability to modulate the neuronal toxicity of ApoE fragments selected from the fragments represented by SEQ ID NO: 2 and SEQ ID NO: 3.

In preferred embodiments, the methods are carried out so as to screen for pharmacological agents having the ability to decrease the neuronal toxicity of ApoE fragments selected from the fragments represented by any one of SEQ ID NOs: 1, 2 or 3. The methods may be carried out so as to screen for pharmacological agents having the ability to decrease the neuronal toxicity of ApoE fragments selected from the fragments represented by SEQ ID NO: 2 and SEQ ID NO: 3.

The methods of screening for pharmacological agents having the ability to modulate neuronal toxicity comprise a step of contacting a neural cell or non-human animal with a candidate pharmacological agent in the presence of at least one ApoE fragment and measuring or detecting the resultant toxicity.

For embodiments wherein the candidate pharmacological agent is contacted with neural cells so as to assess neurotoxicity, the assay may typically be performed *in vitro* using a neural cell culture. The neural cells are preferably neuronal cells. The cells may represent primary neuronal cells, for example, a rat hippocampal cell culture. Alternatively or in addition, the neural cells or neuronal cells may represent an established cell line, for example a neuroblastoma line such as Neuro2A or N2a cells.

The ApoE fragment may be present in the neural cell culture as a result of exogenous administration to the cells, for example administration via the cell culture medium. Alternatively or in addition, the ApoE fragment may be present as a result of recombinant expression of the ApoE fragment by the neural cells of the culture. More specifically, the neural or neuronal cells of the culture may have been engineered so as to recombinantly express an Apo fragment as represented by any one of SEQ ID NOs: 1-3, and the effects of a candidate pharmacological agent may be assessed using the cells expressing the fragment.

The ability of the candidate pharmacological agent to modulate, for example decrease, the neurotoxic effects of the ApoE fragments may be assessed by any suitable assay technique. Techniques for monitoring cell death are known to those skilled in the art and may be used to detect neuronal cell death as a measure of neuronal toxicity. Neurotoxicity may also be assessed using any of the exemplary techniques or assays described herein. For example, neurotoxicity may be detected or monitored indirectly by measuring cellular metabolism. Mitochondrial respiration may be measured in accordance with the technique described in Example 7.

For the purposes of assessing the ability of the candidate pharmacological agent to modulate the neuronal toxicity of the ApoE fragments, the effect seen in the presence of the candidate pharmacological agent may be compared to a control. The control may simply be the neural or neuronal cell culture in the absence of any candidate pharmacological agent. Alternatively, neurotoxicity may be measured for a neuronal cell culture exposed to a control pharmacological agent that is known to have no effect on ApoE fragment-induced toxicity. In certain embodiments, the effect of a candidate pharmacological agent may be determined alongside a control pharmacological agent that is known to decrease or inhibit the neurotoxic effects of ApoE fragments. In such embodiments, the candidate pharmacological agent may be assessed for efficacy relative to the agent that is known to decrease or inhibit the neurotoxic effects of ApoE fragments.

For embodiments wherein the candidate pharmacological agent is contacted with a non-human animal so as to assess neurotoxicity, the pharmacological agent may be administered to the non-human animal via any suitable route of administration. The non-human animal may be selected from a mouse, rat, rabbit, or any other suitable experimental animal. The ApoE fragment may be provided to the non-human animal prior to or concurrently with the pharmacological agent. Alternatively, the non-human animal may have been genetically engineered so as to recombinantly express the neurotoxic ApoE fragments. For example, the experimental animal may recombinantly express the neurotoxic ApoE fragments in the brain such that the effect of the candidate pharmacological agent on neurotoxicity can be determined.

For embodiments wherein the candidate pharmacological agent is tested in a non-human animal, the effect of the candidate agent may be determined by any suitable assay technique for the measurement of neurotoxicity. In certain embodiments, neurotoxicity is assessed by *in vivo* imaging of the brain of the animal. Alternatively or in addition, the animal may be sacrificed at the end of a testing period and the brain tissue examined for evidence of neurotoxic effects. Suitable controls may be employed as described above for the *in vitro* assays.

The methods of screening described herein may lead to selection of a particular pharmacological agent having the ability to modulate the neurotoxicity of ApoE fragments. For example, a pharmacological agent may be selected if it is found to decrease or inhibit the neurotoxicity of one or more ApoE fragments described herein by at least 10%, at least 20%, at least 50%, at least 80% or at least 90%.

In a further aspect, provided herein are methods of screening for pharmacological agents having the ability to modulate the production of ApoE fragments wherein the ApoE fragments are selected from the fragments represented by any one of SEQ ID NOs: 1, 2 or 3. In certain embodiments, the methods involve screening for pharmacological agents having the ability to modulate the production of ApoE fragments selected from the fragments represented by SEQ ID NO: 2 and SEQ ID NO: 3. In certain embodiments, the methods are carried out so as to screen for pharmacological agents having the ability to inhibit the production of ApoE fragments selected from the fragments represented by any one of SEQ ID NOs: 1, 2 or 3. In certain embodiments, the methods are carried out so as to screen for pharmacological agents having the ability to inhibit the production of ApoE fragments selected from the fragments represented by SEQ ID NO: 2 and SEQ ID NO: 3.

The methods comprise contacting a neural cell expressing apolipoprotein E with a candidate pharmacological agent and detecting the amount of the fragment produced. The methods may typically comprise contacting a neural cell population expressing apolipoprotein E with a candidate pharmacological agent and detecting the amount of the ApoE fragment produced by the population. The amount of ApoE fragment may typically be measured after a defined period of time during which the candidate pharmacological agent is contacted with the neural cell population.

In certain embodiments, the neural cell expressing apolipoprotein E is contacted with the candidate pharmacological agent *in vitro.* In such embodiments, the candidate pharmacological agent may be applied to a neural cell culture. The neural cells of the culture may be neuronal cells and may be primary neuronal cells or neuronal cell lines as described above.

In certain embodiments, the neural cell expressing apolipoprotein E may be contacted with the candidate pharmacological agent *in vivo.* In such embodiments, the candidate pharmacological agent may be administered to an animal, preferably a non-human animal, having neural cells expressing apolipoprotein E and the amount of ApoE fragment produced by the neural cells *in vivo* may be detected. The pharmacological agent may be administered to the animal via any suitable route of administration. The amount of ApoE fragment produced in the presence of the pharmacological agent may be detected by *in vivo* imaging of the animal, for example imaging of the brain of the animal. Alternatively or in addition, the amount of ApoE fragment produced may be detected in a sample obtained from the animal such that the detection step is performed *in vitro.* The sample obtained from the animal may be any sample suspected of containing ApoE fragments, for example brain tissue or cerebrospinal fluid.

The candidate pharmacological agent's ability to modulate or inhibit the production of ApoE fragments may be determined based upon a comparison with a control. For example, the amount of ApoE fragment measured in the presence of the candidate pharmacological agent may be compared with the amount of ApoE fragment measured in a control neural cell population expressing apolipoprotein E wherein the control neural cell population has not been exposed to any pharmacological agent. Alternatively, the control neural cell population may be treated with a control pharmacological agent that is known not to affect the production of ApoE fragments.

The amount of the ApoE fragment produced by the neural cell population may be determined at the mRNA or protein level. Suitable techniques for the detection/quantitation of transcriptional products and suitable techniques for assessing protein levels are known in the art. For example, the mRNA levels of the ApoE fragment may be determined by hybridisation techniques, such as Northern blotting or microarray technologies, and/or amplification-based techniques such as RT-PCR or nucleic-acid sequence-based amplification (NASBA). The protein levels of the ApoE fragment may be determined by immunoassay techniques such as immunoblot analysis, ELISA, radioimmunoassay, Elispot etc.

In certain embodiments, the neural cell or cells contacted with the candidate pharmacological agent express a full-length apolipoprotein E protein, preferably a full-length human apolipoprotein E protein. In preferred embodiments, the neural cells express full-length human ApoE4. The neural cells may have been genetically modified so as to recombinantly express the apolipoprotein E protein. For embodiments wherein the neural cells express the full-length apolipoprotein E protein, the methods described herein can be used to screen for pharmacological agents having the ability to inhibit transcription, translation and/or secretion of full-length apolipoprotein E and also pharmacological agents having the ability to inhibit post-translational processing of apolipoprotein E into the neurotoxic ApoE fragments described herein. In certain embodiments, the screening methods described herein screen for pharmacological agents having the ability to inhibit the processing or cleavage of full-length apolipoprotein E into neurotoxic ApoE fragments.

In certain embodiments, the neural cell or cells contacted with the candidate pharmacological agent express an apolipoprotein E fragment as described herein. The neural cells may have been genetically modified such that they express recombinant ApoE fragments in addition to or as an alternative to full-length apolipoprotein E. For embodiments wherein the neural cells express the ApoE fragment, the methods described herein can be used to screen for pharmacological agents having the ability to inhibit direct expression of such neurotoxic fragments.

The pharmacological agents for testing in any of the screening methods described herein may be selected from any class of agent. Pharmacological agents that may be tested in accordance with the methods include but are not limited to small molecules, organic or inorganic molecules, biological molecules including antibodies and antigen binding fragments thereof, natural or synthetic polypeptides or peptides, nucleic acid therapeutic agents including antisense RNA species and double-stranded RNA species for use as RNA interfering agents, for example siRNA molecules.

Pharmacological agents identified by the methods of screening described herein may be useful as agents for the prevention and/or treatment of subjects having neurological diseases or conditions associated with cognitive decline as defined elsewhere herein. The pharmacological agents may be used to treat neurodegenerative diseases or disorders. In certain embodiments, the pharmacological agents identified by the methods of screening described herein may be used to prevent or treat mild cognitive impairment (MCI) or Alzheimer's disease (AD).

### Methods of detection

In a further aspect, provided herein are methods for detecting the presence or amount of an apolipoprotein E (ApoE) fragment consisting of the amino acid sequence of any one of SEQ ID NOs: 1-3 in a subject. In certain embodiments, the methods are for detecting the presence or amount of an ApoE fragment consisting of the amino acid sequence of SEQ ID NO: 1. In certain embodiments, the methods are for detecting the presence or amount of an ApoE fragment consisting of the amino acid sequence of SEQ ID NO: 2. In certain embodiments, the methods are for detecting the presence or amount of an ApoE fragment consisting of the amino acid sequence of SEQ ID NO: 3.

The methods comprise contacting a sample obtained from the subject with an aptamer that binds to the fragment thereby detecting the presence or the amount of the ApoE fragment in the sample. The methods are carried out *in vitro.*

The sample obtained from the subject may be any sample expected to contain one or more ApoE fragments. The sample may be taken from blood e.g. serum, peripheral blood, whole blood or whole blood pre-treated with an anticoagulant such as heparin, plasma or serum. The sample may be obtained from the region of the brain or central nervous system of the subject including the cerebrospinal fluid.

The presence or amount of the ApoE fragment in the sample obtained from the subject is detected by contacting the sample with an aptamer. As used herein, the term "aptamer" refers to a single-stranded oligonucleotide (DNA or RNA) that exhibits binding specificity for a particular target, in this case one or more ApoE fragments as described herein. Aptamers for use in the methods of detection described herein may possess any oligonucleotide sequence or tertiary structure provided that they specifically bind to at least one ApoE fragment as described herein. As used herein, the term "specifically bind" refers to the ability of a molecule (an aptamer) to preferentially bind to a given target.

The binding between the aptamer and the ApoE fragment in the sample may be measured by any suitable technique so as to determine the presence or amount of ApoE fragment in the sample.

In certain embodiments, the sample is obtained from a subject having or suspected of having a neurological disease or disorder, for example a neurodegenerative disorder. In certain embodiments, the sample is obtained from a subject having or suspected of having MCI or AD. The subject may have been previously diagnosed with a neurological or neurodegenerative disease or disorder, for example Alzheimer's disease. Alternatively or in addition, the subject may be receiving treatment or have received treatment for a neurological or neurodegenerative disease or disorder, for example Alzheimer's disease.

The method according to this aspect of the disclosure may be carried out so as to detect, diagnose or assist with the diagnosis of a neurological or neurodegenerative disease in the subject. For example, the method may be carried out so as to detect, diagnose or assist with diagnosis of Alzheimer's disease.

For embodiments wherein the amount of ApoE fragment is detected so as to diagnose or assist with diagnosis of disease, the amount of ApoE fragment in the sample may be compared with a pre-determined threshold value or cut-off so as to assess the likelihood of disease in the subject. For example, the pre-determined threshold value or cut-off may have been or be determined based upon the levels of the corresponding ApoE fragments detected in a cohort of healthy subjects. If the amount of ApoE fragment in the sample obtained from the subject exceeds the pre-determined threshold value for the cohort of healthy subjects, the subject may be diagnosed as having disease, for example Alzheimer's disease.

In certain embodiments, the ApoE fragments may be detected in a sample obtained from a subject so as to monitor the subject's clinical response to treatment. The treatment may be treatment for any neurological or neurodegenerative disorder but is preferably treatment for Alzheimer's disease. A decline in the level of ApoE fragments measured in multiple samples obtained from the subject over a period of time, for example a period of time coinciding with a course of treatment, may be indicative of a clinical response to treatment.

### Incorporation by Reference

Various publications are cited in the present application, each of which is incorporated by reference herein in its entirety.

### Examples

The invention will be further understood with reference to the following nonlimiting examples.

### Example 1

### Analysis of ApoE fragments in human brain extracts from Alzheimer's disease patients and controls

This example describes the homogenization of human brain tissues and the following Western blot analysis of ApoE fragments from brain extracts in Radio-Immunoprecipitation Assay (RIPA) buffer with 2% sodium dodecyl sulfate (SDS).

### Materials and methods

Brain tissue homogenization and sample preparation: Fresh frozen human brain tissue from Alzheimer's disease (AD) patients (n=24) and controls (n=14), with various APOE genotypes, were homogenized by 1:5 weight:volume in RIPA 2% SDS extraction buffer followed by a 16000 x *g* centrifugation for 1 h. The resulting supernatant was frozen at -80°C until analysis.

Analysis of ApoE fragments in human brain extracts: RIPA 2% SDS brain extract containing 10 µg total protein was mixed with 2x Laemmli sample buffer, boiled for 5 min at 95°C and loaded onto SDS-PAGE gels (Bolt™ 12% Bis-Tris Plus 10 well, Thermo Fisher). Gels were run for 30-40 min at 180 V, after which proteins were transferred from the gels to nitrocellulose membranes using the Trans-Blot® Turbo™ system (BioRad). Membranes were blocked in Odyssey® blocking buffer for 1 h and then incubated over night at RT with a polyclonal anti-ApoE antibody (Calbiochem, cat. No. #178479) diluted 1:2000 in Odyssey® blocking buffer with 0.1% Tween® 20. Membranes were washed and incubated for 1 h at RT with detection antibody anti-goat-800CW (LI-COR, cat. No 925-32214) diluted 1:25000 in Odyssey® blocking buffer with 0.1% Tween® 20. Membranes were washed and images acquired using Odyssey® FC (LI-COR). Image Studio Software (version 5.2) was used to quantify the relative amount of ApoE fragments in ratio to the amount of full-length ApoE in the acquired Western blot images.

### Results

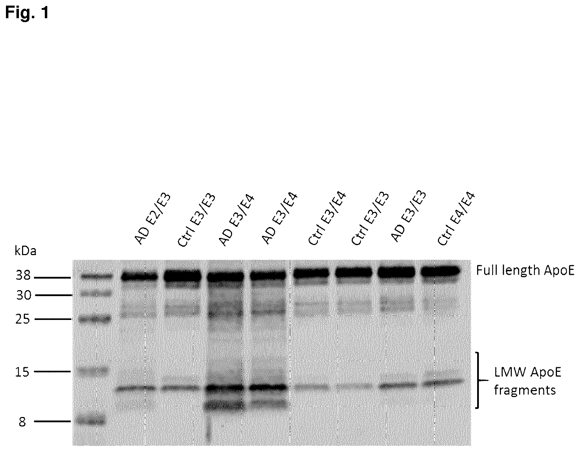

Full-length ApoE as well as several low molecular weight (LMW) ApoE fragments were identified by Western blot analysis of human brain RIPA 2% SDS extracts (n=38). Figure 1 shows a representable membrane from Western blot analysis. The LMW ApoE fragments were estimated to be 10, 12, 14-15 and 17 kDa in size (Figure 2).

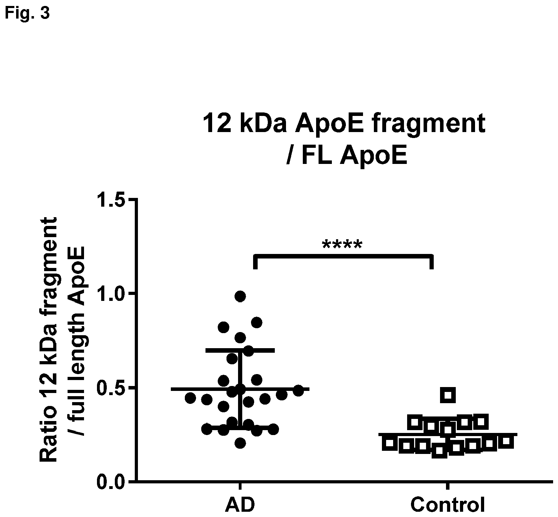

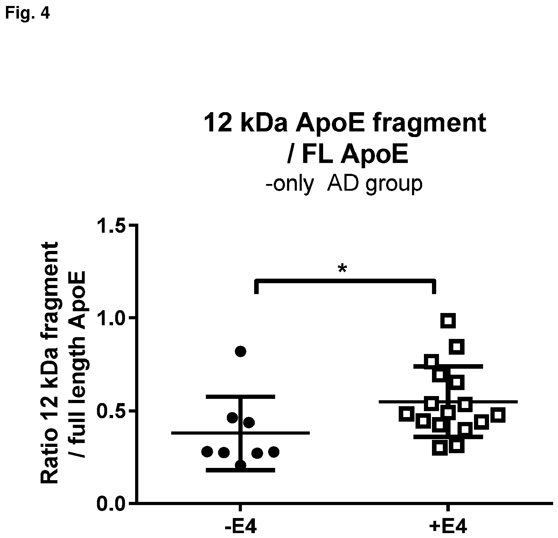

Analysis of ApoE fragments, in ratio to full-length (FL) ApoE, demonstrated that the 12 kDa ApoE fragment was significantly incraesed in the AD group (n=24) as compared to the Control group (n=14), see Figure 3. In addition, a significant increase of the 12 kDa ApoE fragment was observed in APOE ε4 carriers in the AD group (Figure 4).

### Example 2

### Extraction and isolation of ApoE fragments from human brain extracts from Alzheimer's disease patients

This example describes a procedure for isolation and concentration of full-length ApoE and 12 and 15 kDa ApoE fragments from human brain extracts, in order to prepare pure samples of ApoE with a protein concentration sufficient for amino acid sequence analysis.

### Materials and methods

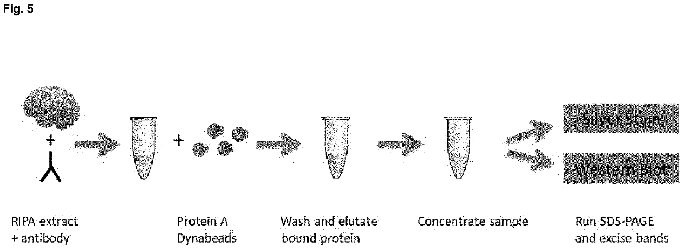

Isolation of ApoE from human brain extracts from AD patients with various APOE genotypes: A protocol for immunoprecipitation (IP) of ApoE from human brain extracts was established. Protocol optimization resulted in pure samples of ApoE with a protein concentration sufficient for amino acid sequence analysis. For a schematic overview of the workflow, see Figure 5. Human brain RIPA 2% SDS extracts, with a total protein concentration of 1.5 mg, were mixed with IP buffer (1xPBS, 0.05% Tween® 20, 0.1% Triton X-100, protease inhibitor cocktail) and ApoE was immunoprecipitated by adding 200 µg of an anti-ApoE C-terminal antibody, with a binding epitope within amino acids 237-299 (Thermo Scientific, cat. No PA5-27088). Complexes between IP antibody and ApoE in the brain extract were allowed to form during an incubation for 2 h at RT with head-over-tail rotation. 500 µl Protein A Dynabeads (Dynal, Thermo Scientific, cat. No 10002D) were added to the IP mixture and incubated for 1 h at RT with head-over-tail rotation, after which the Protein A Dynabeads were washed to remove unspecific binding to the beads. ApoE proteins bound to the Protein A Dynabeads (via the IP antibody) were eluted in 250 µl elution buffer (1.25 mM Tris pH 6.8, 0.005% SDS) and incubated for 5 min at 95°C with shaking at 900 rpm. After a quick spin, the samples were placed on the DynaMag™-2 magnet and the liquid was transferred to a new tube.

Concentration of isolated ApoE followed by analysis by SDS-PAGE: In order to concentrate the ApoE protein, the eluted IP sample was centrifuged in a rotational vacuum concentrator at 1300 rpm at 40°C for approximately 2 h, to reduce the volume from 250 µl to approximately 15 µl. 2x Laemmli buffer was added to the concentrated samples and the samples were incubated for 5 min at 95°C with 900 rpm. After a quick spin, the samples were loaded onto SDS-PAGE gels (Bolt™ 12% Bis-Tris Plus 10 well, Thermo Fisher, cat. No NW04120BOX). Gels were run for 30-40 min at 180 V, after which one gel was used for confirmation of ApoE fragments by Western blot analysis and one gel was silver stained and used for excision of ApoE.

Western blot analysis of SDS-PAGE gels: Proteins were transferred from the gels to nitrocellulose membranes using the Trans-Blot® Turbo™ system (BioRad). Membranes were blocked in Odyssey® blocking buffer for 1 h and then incubated over night at RT with the anti-ApoE C-terminal antibody (Thermo Scientific, cat. No PA5-27088) diluted 1:2000 in Odyssey® blocking buffer with 0.1% Tween® 20. Membranes were washed and incubated for 1 h at RT with detection antibody anti-rabbit-800CW (LI-COR, cat. No 925-32211) diluted 1:25000 in Odyssey® blocking buffer with 0.1% Tween® 20. Membranes were washed and images acquired using Odyssey® FC (LI-COR).

Silver staining of SDS-PAGE gels: Gels were fixated and stained with silver staining according to manufacturer's instructions (Pierce Silver Stain for Mass Spectrometry, Thermo Scientific, cat. No 24600). After the silver staining was complete, the stop buffer was exchanged to Milli-Q H₂O and rinsed 2x 10 min. Full-length ApoE, and the 12 and 15 kDa ApoE bands were excised from the gel and placed in Milli-Q H₂O in clean Eppendorf tubes.

### Results

Using the established IP protocol (Figure 5), ApoE was isolated from human AD brains with various APOE genotypes (ε2/ε3, ε3/ε3, ε3/ε4 and ε4/ε4), and the eluted proteins were run on SDS-PAGE.

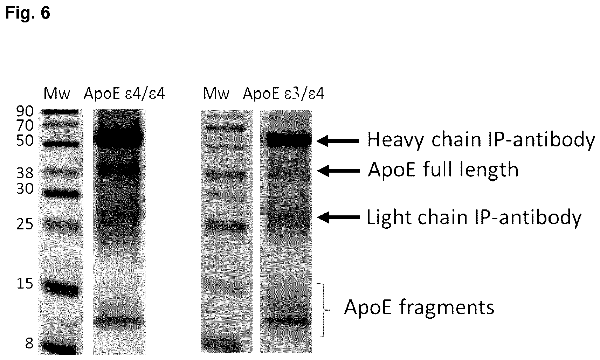

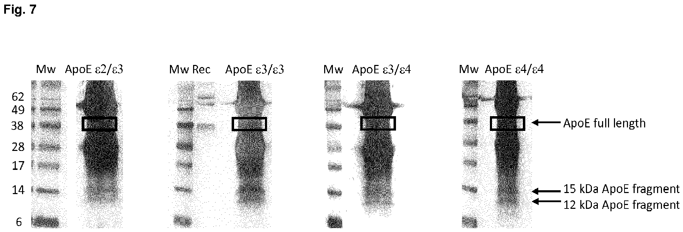

Extraction of ApoE was confirmed by Western blot analysis. Figure 6 shows a representative Western blot membrane demonstrating several bands with ApoE fragments, as well as full-length ApoE. In addition, isolated and concentrated ApoE proteins were stained by silver staining of the SDS-PAGE gels as shown in Figure 7. ApoE fragments of approximately 12 and 15 kDa in size were visualized and excised from the silver stained gels. As reference samples, recombinant full-length ApoE protein and full-length ApoE from the human brain IP sample were also excised from the silver stained gels.

### Example 3

### Identification of trypsin cleavage sites in 12 kDa ApoE fragment

### Sample preparation

Silver-stained strips of gels from Example 2 in 1.5 ml PP-tubes, including a band of recombinant human full-length ApoE4 (rhApoE4) and/or 34 kDa from immunoprecipitation, band of 15 kDa from immunoprecipitation, and band of 12 kDa from immunoprecipitation, were washed with enough water and followed by dehydration using 500 µl acetonitrile (ACN; from Wako). After turning each gel white, any solvent was removed and followed by adding 500 µl of water to get each gel swelling. After removal of water, 500 µl of Silver Quest Destainer (Invitrogen) was added to each gel and incubated for 15 min at room temperature. After removal of any destainer solvent, 1000 µl of water was added, then incubated for 10 min at room temperature. After the removal of water, 1000 µl of water was added again to wash each gel, then any solvent was removed from tubes. 500 µl ACN was added to each gel, then excess ACN was removed after turning each gel white.

500 µl of 10 mM dithiothreitol (DTT; from Wako) was added into gels, followed by incubation at 56°C for 30 min. After removal of DTT solution, 500 µl ACN was added to shrink each gel with gentle mixing incubation at room temperature for 10 min. After removal of ACN, 55 mM iodoacetoamide (IAA; from Wako) was added into each tube, then incubated at room temperature in the dark for 30 min. After removal of IAA solution, 500 µl ACN was added into each tube again, with occasional vortex mixing for 10 min, in order to obtain shrunk gels. After removal of ACN, 300 µl of 13 µg/ml trypsin in 10 mM ammonium bicarbonate with 10% ACN was added into the gels, then incubated at 5°C for 6 hours. Then, gels were placed in a 37°C chamber to promote digestion of proteins in each gel, followed by incubation overnight.

600 µl of 5% formic acid in water / ACN in a 1/2 (v/v) solution was added to each tube and mixed well with vortex. Then, incubation at 37°C with gentle rotating was conducted to obtain a solution including tryptic peptides from each gel. The obtained solution was dried by SpeedVac system (Thermo Fisher Scientific), followed by reconstitution using 300 µl of 5% methanol in 0.1% TFA-water. The solution was desalted by Monospin C18 solid extraction column (GL Sciences) according to the vendor's instruction manual, after which the eluent was dried by SpeedVac system. 30 µl of 5% methanol in 0.1% TFA-water was added into each tube to obtain the final reconstituted solution. The solution was subjected to LC-MS analysis.

### LC/MS analysis

The obtained samples were analyzed in a nano-flow LC-MS/MS system using a Q Exactive HF mass spectrometer (Thermo Fisher Scientific) coupled with an online UltiMate 3000 Rapid Separation LC (Dionex) and an HTC PAL sample injector (CTC Analytics) fitted with a microcapillary column (360 nm outer diameter (OD) × 100 µm ID), which was packed with < 20 cm of ReproSil C18-AQ 3 µm beads (Dr. Maisch GmbH) and equipped with an integrated electrospray emitter tip (P-2000 laser-based puller, Sutter Instruments). Each sample was loaded onto the capillary column by 4 µl full-loop mode injection. For LC separation, a mobile phase A of 4% ACN and 0.5% acetic acid (Wako) and a mobile phase B of 80% acetonitrile and 0.5% acetic acid were used for multiple linear gradient elution from 1-40% of B over 60 min, 40-70% of B over 10 min, and 70-99% of B over 5 min, and then held at 99% of B for 10 min at 500 nl/min. The total analysis time for each sample was 120 min.

Each sample was analyzed using data dependent analysis (DDA) mode, which used higher energy collision dissociation (HCD) MS/MS scans (resolution 30000) for the top 15 most abundant ions of each full-scan MS from m/z 300 to 3000 (resolution 60000) with a full-scan MS ion target of 3 × 10° ions and an MS/MS ion target of 2 × 10⁵ ions. The maximum ion injection time for the MS/MS scans was 100 ms. The HCD normalized collision energy was set to 27, the dynamic exclusion time was set to 20 s, and the peptide match and isotope exclusion functions were enabled.

### Data analysis

All DDA mass spectra were analyzed with Proteome Discoverer ver. 2.1 (Thermo Fisher Scientific) using a human ApoE4 FASTA file. SEQUEST-HT algorithm was used for MS/MS searching of the data sets with the following parameters: oxidation of methionine as variable modifications, carbamidomethylation of cysteine as a fixed modification, and trypsin as the digestion enzyme. Two missed cleavages per peptide were allowed. The mass tolerance for precursor ions was set to 10 ppm, and the mass tolerance for product ions was set to 20 mDa. A maximum false discovery rate (FDR) of 1% was applied for peptide identification. Protein identification required more than two peptides per protein. Then, a detailed analysis focusing only on ApoE4 was conducted to identify the cleavage sites of the 12 kDa band (ApoE4 fragment).

### Results

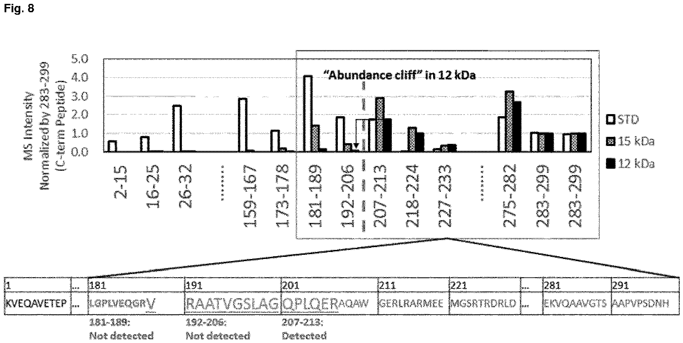

The 12 kDa ApoE fragment was subjected to tryptic digestion to survey the cleavage sites of ApoE on a peptide basis. rhApoE4 and 15 kDa bands were analyzed as references. The results (Figure 8) showed there was an "abundance cliff" in the tryptic peptides from the 12 kDa band between a peptide corresponding to amino acid residues 192-206 of ApoE and a peptide corresponding to amino acid residues 207-213. This means that there is at least one cleavage site in the region from amino acid residue 190 to amino acid residue 206, because the "207-213 peptide" was clearly detected with high MS intensity. Short peptides (less than 5 residues of amino acids) were eliminated from the analysis, so e.g. the VR dipeptide at positions 190-191 was not observed.

### Example 4

### Identification of LysC cleavage sites in 12 kDa ApoE fragment

### Materials and methods

Sample preparation, LC/MS analysis and data analysis were performed as described above for Example 3.

### Results

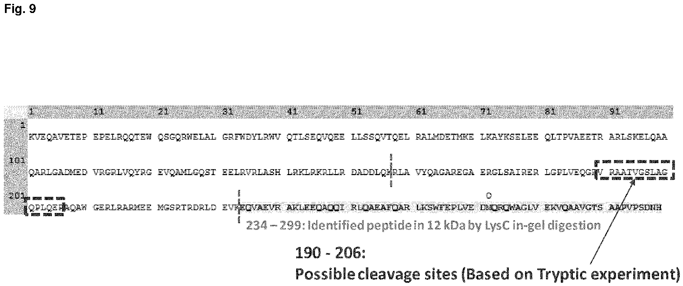

To narrow down the cleavage site of 12 kDa ApoE fragment on an amino acid basis, digestion by another enzyme, lysyl endopeptidase (LysC), was carried out. As a result of standard LysC proteomic analysis of the 12 kDa band (fixed cleavage at lysine C-terminal), the only peptide detected was a peptide corresponding to amino acid residues 234-299 of ApoE (Figure 9). This confirms the result of Example 3, to the effect that there is at least one cleavage site between positions 190-206. Notably, a peptide corresponding to amino acid residues 158-233 of ApoE was detected upon cleavage of rhApoE4 (not shown), but was not detected when cleaving the 12 kDa band, further supporting the existence of at least one cleavage site between positions 190-206.

### Example 5

### Further characterization of LysC cleavage sites in 12 kDa ApoE fragment

### Materials and methods

Sample preparation and LC/MS analysis were performed as described above for Example 4. Data analysis was performed as described above for Example 4, except that target analysis (describing peaks and the integration) from extracted-ion chromatograms (XIC) was performed for the specific peptides cleaved at unexpected regions. This peak qualification analysis was conducted by Qual Browser in Xcalibur 4.0 software (Thermo Fisher Scientific).

### Results

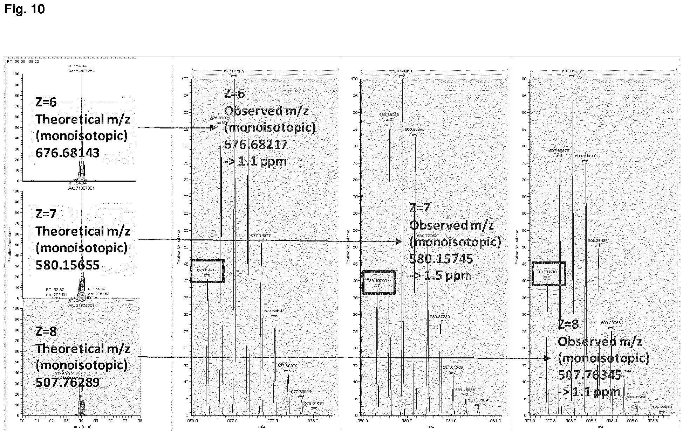

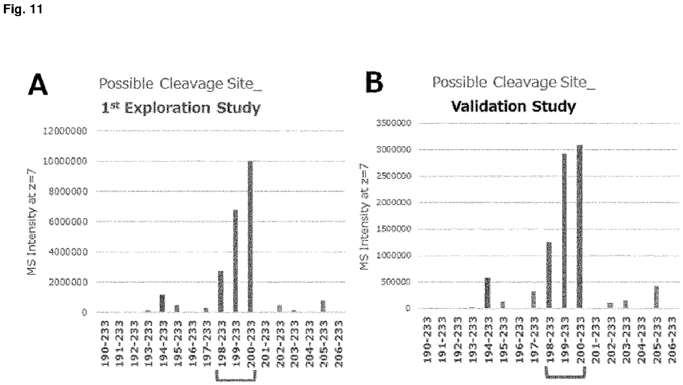

Prior to the detailed analysis of possible cleavage sites that give rise to the identified 12 kDa fragment, it was investigated whether the peptide corresponding to amino acid residues 158-233 of ApoE (RLAVYQAGAR EGAERGLSAIR ERLGPLVEQG RVRAATVGSL AGQPLQERAQ AWGERLRARM EEMGSRTRDR LDEVK) obtained by LysC digestion was detected in any of the rhApoE4 band, the 34 kDa band from immunoprecipitation, and the 12 kDa band from immunoprecipitation. This was done by describing each XIC with the theoretical m/z (z=10-15, 5 ppm mass tolerance). The results showed that the 158-233 peptide was clearly detected in the solution from rhApoE4 and the 34 kDa band, which means that there is no artifact cleavage in the sample preparation step. On the other hand, the 158-233 peptide was not observed in the sample solution from the 12 kDa band. That indicated that there is at least one cleavage site between 158-233 in the 12 kDa ApoE4 fragment. In summary, the LC/MS results from the tryptic process described in Example 3 elucidated the preliminary cleavage site between positions 190-205, then the site was confirmed by the LysC process as described in Example 4 and above. To narrow down the possible cleavage sites between 190-205 on an amino acid basis, all theoretical "non-conventional" peptides provided by LysC digestion of the 12 kDa band (i.e. 190-233, 191-233, 192-233, 193-233, 194-233, 195-233, 196-233, 197-233, 198-233, 199-233, 200-233, 201-233, 202-233, 203-233, 204-233, 205-233, and 206-233) were searched by describing each XIC to check whether the fragment peak was detected or not. Figure 10 shows an example of the results, when looking for "non-conventional LysC peptide" corresponding to amino acid residues 200-233 of ApoE (GQPLQERAQA WGERLRARME EMGSRTRDRL DEVK; [M]= 4054.04490). The theoretical monoisotopic m/z values (charges 6, 7 and 8) for the 200-233 peptide are 676.68143, 580.15655 and 507.76289, respectively. The extracted chromatogram for each m/z value provides a single peak at the same retention time, and the observed masses agree with the theoretical in each case with a mass accuracy of less than 2 ppm. These results strongly reinforced that non-conventional LysC peptides had been identified, leading to a positive identification of the specific cleavage sites that yield the 12 kDa ApoE fragment (Figure 11A). A duplicate experiment on another sample (ApoE e3/e4 allele) showed reproducible results (Figure 11B), confirming the determination of the cleavage sites.

In conclusion, nanoLC-MS/MS analysis of brain samples from three individual donors (ApoE ε3/ε4) demonstrated that the major cleavage sites that yield the 12 kDa ApoE fragment were at the N-terminus of 198L, 199A and 200G (Figure 11).

### Example 6

### Identification of cleavage sites in 12 kDa ApoE fragment in human brains with ε4/ε4, ε2/ε3 and ε3/ε3 alleles

### Materials and methods

Sample preparation, LC/MS analysis and data analysis were performed as described above for Examples 3-5.

### Results

The N-termini 198L, 199A and 200G were identified as the main cleavage sites to yield the 12 kDa ApoE fragment from ApoE ε3/ε4. To clarify if these cleavage sites are specific only to the ε4 allele and not ε2 or ε3, 12 kDa bands from the brains of ApoE ε4/ε4, ε2/ε3 and ε3/ε3 carriers were analyzed by means of the same manner as the previous section.

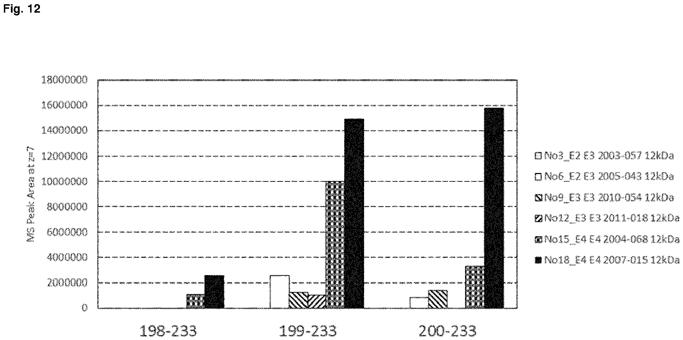

The results are presented in Figure 12 and showed that ε4/ε4 carriers exhibited the expected cleavages at the N-terminus of 198L, 199A and 200G (mainly 199A and 200G), whereas ε2/ε3 and ε3/ε3 carriers showed considerably lower signal of the sites cleavages than ε4/ε4 carriers. That results indicated the cleavage at the N-terminus of 198L, 199A and 200G are more abundant in ε3/ε4 and ε4/e4 allele carriers.

### Example 7

### Neuronal toxicity of identified ApoE fragments

### Materials and methods

Cell culture: Neuro2A cells (ATCC) were seeded at 5.0 × 10⁴ cells/well in a 24 well plate (Falcon) and cultured in D-MEM High Glucose (WAKO) containing 10% fetal bovine serum. Transfection of pAAV-CMV vectors encoding human ApoE4 (full-length) or the identified ApoE fragments (198-299, 199-299, 200-299) was done using Lipofectamine LTX and Plus Reagent (Invitrogen) on 1 day after seeding. 2 days later, vector-transfected cells were collected for Western blot analysis or seeded again at 2.0 × 10⁴ cells/well in a Seahorse XF96 cell culture microplate (Agilent Technologies) 4 hours before mitochondrial respiration measurement.

For assays using rat hippocampal neurons, the dissected hippocampi from fetuses obtained on embryonic day (E) 18 from timed pregnant Wistar rats (Charles River Laboratories) were digested using trypsinization and mechanical dissociation. The dissociated neurons were seeded at 1.5 × 10⁴ cells/well in Seahorse XF96 cell culture microplate (Agilent Technologies) for mitochondrial respiration measurement or 1.0 × 10⁵ cells/well in 24-well plate (Falcon) for Western blot analysis. Infection of AAV6 with full-length human ApoE4 or identified ApoE fragments (198-299, 199-299, 200-299) was performed at 7 days *in vitro* (DIV). Measurement of mitochondrial respiration or sample collection for Western blot analysis was performed at 7 days after infection (14 DIV).

Western blot analysis: Cells were lysed by RIPA buffer (50 mM Tris-HCI pH 7.6, 5 mM EDTA, 1 mM EGTA, 1% NP40, 0.25% sodium deoxycholate, 0.1 M NaCl, 0.5 mM PMSF) containing complete (EDTA-free) protease inhibitor cocktail (Roche) and PhosSTOP protein phosphatase inhibitor (Sigma), and sonicated. Sample Buffer Solution with Reducing Reagent (6x) (Nacalai Tesque) was added before SDS-PAGE. For SDS-PAGE, XV PANTERA MP Gel (DRC) 15% was used. For transfer, Trans-Blot Turbo (BIO-RAD) was used. For immunoblotting, iBind Western Systems (ThermoFisher Scientific) was used together with the following antibodies: anti-ApoE PA5-27088 (ThermoFisher Scientific); 178479 (Calbiochem).

Mitochondrial respiration measurement: Real-time measurement of oxygen consumption rates (OCR) was performed using an Extracellular Flux Analyzer XFe96 (Agilent Technologies). Before measurement, the culture medium was replaced by 37 °C pre-warmed XF Base Medium (Agilent Technologies) containing 10 mM sodium pyruvate (Sigma), 10 mM D-glucose (Sigma), 2 mM glutamine (Sigma). The pH of the measurement medium was adjusted to 7.4. The culture plates were incubated at 37 °C for 60 min prior to the assay. For analysis of mitochondrial function, XF Cell Mito Stress Test Kit (Agilent Technologies) was used. Following measurement of basal OCR, mitochondrial complex inhibitors were injected sequentially into each cell. The inhibitors were used at the following concentrations: oligomycin 1 µM; carbonyl cyanide 4-(trifluoromethoxy)phenylhydrazone (FCCP) 0.25 µM for Neuro2A cells, 2 µM for rat hippocampal neurons; rotenone/antimycin A 0.5 µM. OCR values were automatically calculated, recorded and plotted by the XFe96 software. Spare respiratory capacity was measured as (FCCP respiration - basal respiration).

### Results

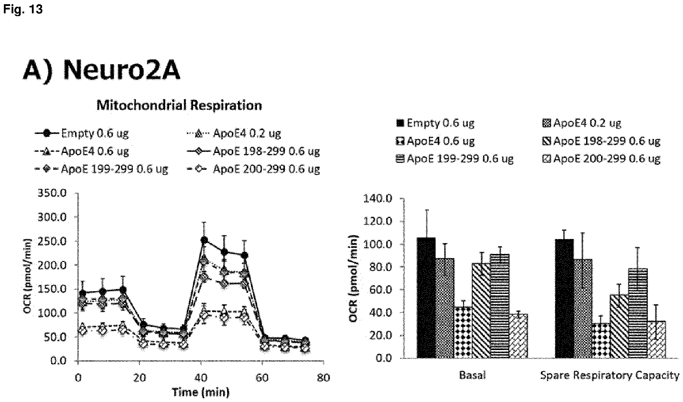

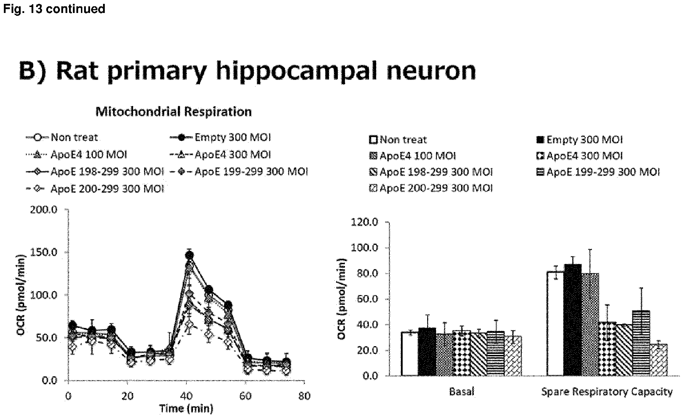

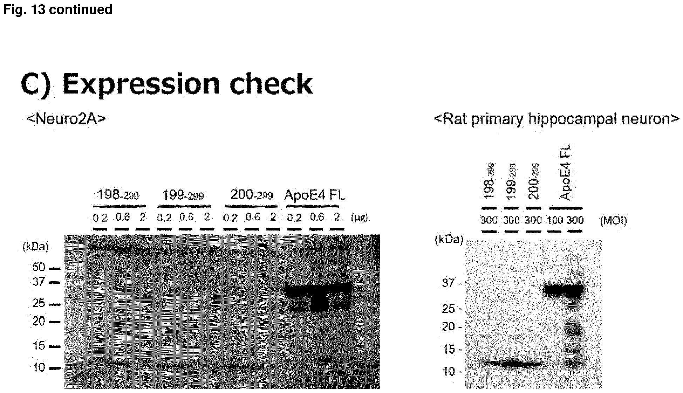

In both Neuro2A cells and rat primary hippocampal neurons, the groups expressing either one of the identified ApoE fragments (198-299, 199-299, 200-299) showed a reduction in spare respiratory capacity (Figure 13A and B), indicating that these fragments inflict mitochondrial damage. In addition, the fragments caused mitochondrial dysfunction at much lower expression levels than did ApoE4 full-length (Figure 13A-C). The results show that the C-terminal fragments of ApoE identified from human brain are neurotoxic.

## Claims

1. A fragment of apolipoprotein E, which consists of an amino acid sequence selected from the group consisting of SEQ ID NO: 2 and SEQ ID NO: 3.

2. The fragment according to claim 1, which consists of the amino acid sequence of SEQ ID NO: 2.

3. The fragment according to claim 1, which consists of the amino acid sequence of SEQ ID NO: 3.

4. The fragment according to any one of the preceding claims, which exhibits neurotoxicity.

5. An isolated nucleic acid encoding the fragment according to any one of claims 1-4.

6. A vector comprising the isolated nucleic acid according to claim 5.

7. A host cell comprising the vector according to claim 6.

8. A transgenic non-human animal comprising the vector according to claim 6.

9. A vaccine comprising an apolipoprotein E fragment consisting of the amino acid sequence of any one of SEQ ID NOs: 1-3.

10. A method of preventing or treating a neurological disease in a subject in need thereof, the method comprising administering to the subject a vaccine in accordance with claim 9.

11. A method of screening for a pharmacological agent having the ability to modulate the neuronal toxicity of an apolipoprotein E fragment consisting of the amino acid sequence of any one of SEQ ID NOs: 1-3,
wherein the method comprises contacting a neural cell or non-human animal with a candidate pharmacological agent in the presence of the fragment and detecting neuronal toxicity or neuronal death.

12. A method of screening for a pharmacological agent having the ability to modulate the production of an apolipoprotein E fragment consisting of the amino acid sequence of any one of SEQ ID NOs: 1-3,
wherein the method comprises contacting a neural cell expressing apolipoprotein E with a candidate pharmacological agent and detecting the amount of the fragment.

13. The method of claim 12, wherein the neural cell expresses the apolipoprotein E fragment.

14. The method of claim 12, wherein the neural cell expresses full-length apolipoprotein E.

15. The method of claim 14, wherein the apolipoprotein E is apolipoprotein E4 (ApoE4).

16. The method of screening according to any one of claims 12-15, wherein the neural cell expressing the apolipoprotein E fragment or full-length protein is a genetically modified cell.

17. A method for detecting the presence or amount of an apolipoprotein E fragment consisting of the amino acid sequence of any one of SEQ ID NOs: 1-3 in a subject,
wherein the method comprises contacting a sample obtained from the subject with an aptamer that binds to the fragment and detecting the presence or the amount of the fragment in the sample.

18. The method according to claim 17, wherein the sample is obtained from a subject having or suspected of having Alzheimer's disease (AD) or mild cognitive impairment (MCI).

19. The method according to claim 17 or claim 18, wherein the presence or amount of the apolipoprotein E fragment is used to detect, diagnose or assist with diagnosis of AD or MCI.
